# EUROPEAN PATENT APPLICATION

(11) **EP 2 898 825 A1**
(43) Date of publication of application: **29.07.2015**
(21) Application number: 14152141.9
(22) Date of filing: 22.01.2014
(51) Int. Cl.: A61B 5/024, A61B 5/00

(54) **Portable device for optical heart rate measurement and method**

(71) Applicant: ams AG, 8141 Unterpremstätten (AT)
(72) Inventor: Enenkel, Jan, 8101 Gratkorn (AT); Manninger, Mario, 8141 Unterpremstätten (AT)
(74) Representative: Epping - Hermann - Fischer

(57) **Abstract**

In one embodiment a portable Device (10) for optical heart rate measurement has a first light-emitting diode (TL1), LED, for generating a light signal (Sl) having a predetermined wavelength, a detector (OR) for detecting a reflected signal (Sr1), the reflected signal (Sr1) depending on a reflection of the light signal (Sl) off a blood vessel of a user of the device (10), a stress sensor unit (PSen) for providing a pressure signal (Sp) as a function of a pressure between the portable device (10) and its user, and a control unit (CTL) connected to the first LED (TL1), the stress sensor unit (PSen) and to the detector (OR), the control unit (CTL) having a first output (Out1) for providing a heart rate signal (Sh). Therein the heart rate signal (Sh) is provided as a function of the reflected signal (Sr1) and the pressure signal (Sp) sensed by the stress sensor unit (PSen).

## Description

The invention relates to a portable device for optical heart rate measurement and to a method for optical heart rate measurement using the portable device.

Portable devices which measure a user's heart rate by optical means have become widespread, e.g. in sports equipment. Such devices are used in close contact to a user's skin, for instance in the form of a wrist watch. The employed optical measurement is based on sending out light of a certain wavelength and detecting its reflection off a blood vessel of the device's user. The user's heart rate can be calculated from this reflection.

Caused by state of the art measurement techniques, algorithms and mechanical build-ups, known devices show weaknesses in real sports environments. Detection of the heart rate when the user is running, for instance in different environments, or when using weights, is problematic as due to the frequent movements of the user it takes time to adjust the optical measurement parameters in order to achieve a reliable and correct measurement of the heart rate. During the necessary adjustments of parameters, a state-of-the-art device might display an incorrect value for the heart rate. Under heavy training conditions, in which the device might move on its user's body or wrist, the measurement could be permanently wrong. Especially, when the rhythm of a user's movement during a run has a similar frequency to his heart rate, it is very difficult to extract the heart rate measurement by narrow band filtering. Currently, acceleration sensors are used in addition to the optical sensor to correlate motion artefacts with measurements from this acceleration sensor. However, this increases system complexity and costs.

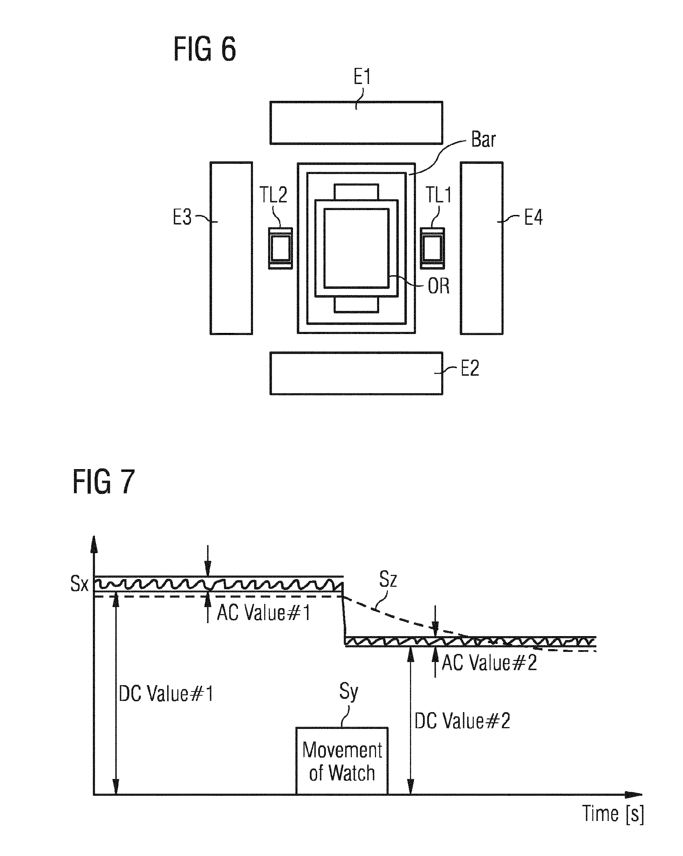

The invention starts out from a device for optical heart rate measurement basically having a light-emitting diode, a photodiode, an acceleration sensor and a micro-controller running a calculation algorithm for the heart rate. The upper part of figure 7 shows a signal Sx detected by such photodiode. In the bottom of figure 7 a signal Sy detected by the acceleration sensor is depicted. The signal Sx from the light sensor has two main components, namely an alternate current component, denominated AC value and a direct current component, denominated DC value. Every time a movement of the device occurs, the signal track might be lost and the algorithm has so search for a new DC value and for a suitable gain for the AC value. In state of the art implementation, this is a "search until it seems to be a heart rate" algorithm. As a consequence, misinterpretation of non-existing signals might arise, e.g. caused by wrongful adjustment of the device, resulting in wrong measurement values. State of the art algorithms additionally show difficulties in differentiating between voltage changes caused by the heart rate and those caused by changes brought about by movement, especially when these frequencies are in the same band.

The objective problem of the present invention can therefore be seen in providing a portable device for optical heart rate measurement and a corresponding method which provide improved results.

The objective is achieved by the subject-matter of the independent patent claims. Embodiments and developments of the invention are defined in the dependent claims.

In one embodiment a portable device for optical heart rate measurement has a first light-emitting diode, LED, a detector, a stress sensor unit and a control unit. The first LED generates a light signal having a predetermined wavelength. The detector is prepared to detect a reflected signal which depends on a reflection of the light signal off a blood vessel of the user of the device. The stress sensor unit is enabled for providing a pressure signal as a function of a pressure between the portable device and its user. The control unit is connected to the first LED, the stress sensor unit and to the detector. The control unit has a first output for providing a heart rate signal. The heart rate signal thereby is provided as a function of the reflected signal and the pressure signal sensed by the stress sensor unit.

The light signal emitted by the first LED is reflected by a blood vessel of the device's user, which reflection is detected in the detector. Concurrently, the stress sensor unit provides the pressure signal indicative of the pressure between the portable device and its user. The control unit calculates the heart rate signal by taking into account the reflected signal and the pressure signal.

The proposed portable device advantageously has the stress sensor unit which provides the pressure signal which is considered in addition to the reflected signal in calculation of the heart rate signal. By this, an increase or a decrease in pressure between the portable device and its user can be measured and calculation of the heart rate signal can be optimized to achieve a more accurate result for the heart rate signal. As an acceleration or gravity sensor is not necessary in the proposed device, system costs is reduced when compared with state of the art implementations.

The pressure thereby represents a mechanical contact pressure between the device and its user. A difference in pressure between the portable device and its user indicates a movement of the part of the body of the user to which the portable device is attached. Consequently, the usage of the stress sensor unit within the portable device enables detection of movements of the user which allows more accurate provisioning of the heart rate signal.

The pressure signal also indicates a distance between the device and its user.

The detector which detects the reflected signal comprises, for example, a photodiode. The control unit comprises, for example, a microcontroller. The control unit runs an algorithm for calculating the heart rate signal.

In a development the wavelength of the light signal is predetermined from a range of 525 to 950 nm in accordance with the skin colour of the device's user.

As light of different colour, i.e. different wavelength, is absorbed differently by different skin colours of users, the wavelength of the light signal is chosen in accordance with the skin colour of the device's user. By this, an optimum reflection of the light signal can be achieved and detected in the reflected signal. The light signal is reflected by small blood vessels which are located in an intersection between dermis and subcutis.

In an example implementation a green LED is used for users with white skin. An infrared LED is employed for users with dark skin.

In an embodiment the stress sensor unit comprises an electrode adapted to be operated as a capacitive sensor in loading mode.

The stress sensor unit when operated as capacitive sensor in loading mode detects a change in capacitance between the electrode and ground potential of the device. Therein, ground potential can be implemented e.g. by an electrode located in the strips of the device.

In an alternative embodiment the stress sensor unit comprises two electrodes adapted to be collectively operated as a capacitive sensor in differential mode.

In this embodiment the mechanical pressure between the device and its user is measured by means of the two electrodes. One electrode is operated as a transmitter; the second electrode is operated as a receiver. A change in capacitance in the electrical field between transmitter and receiver electrode is detected and the pressure signal is provided therefrom.

In another alternative embodiment the stress sensor unit comprises a first and a second pair of electrodes. Each of the two pairs is adapted to be operated as a capacitive sensor in differential mode. The pressure signal is provided as a function of a first signal output by the first pair of electrodes and as a function of a second signal output by the second pair of electrodes.

In each pair, one electrode is operated as a transmitter, whereas the other electrode is operated as a receiver of a capacitive sensor. Consequently, the first pair of electrodes provides the first signal reflecting a change in capacitance between the transmitting and the receiving electrode of the first pair. Likewise, the second pair provides the second signal as a function of a change in capacitance between the transmitting and the receiving electrode of the second pair. First and second signals are combined into the pressure signal.

In yet another alternative embodiment, the stress sensor comprises a resistive sensor.

Instead of a capacitive sensor, this embodiment uses a resistive sensor for detecting a pressure between the portable device and its user. E.g. a piezo or a strain gauge pressure transducer can serve as resistive sensor.

Detection of the mechanical contact pressure between the device and its user improves optical heart rate measurement in the following ways. A person skilled in the art knows that devices currently on the market employ an algorithm run, for instance, in the control unit, which calculates the user's heart rate based on the reflected signal received by the detector. By additionally using the stress sensor unit's pressure signal, the algorithm is enabled to react more quickly to a movement of the device which can easily be detected using the stress sensor.

In more detail, when a movement of the device occurs, the reflected signal has to be re-tracked within the detector. The algorithm consequently has to adapt to new values of this signal, namely to a new DC value and to a suitable gain for the AC value. The additional information provided via the pressure signal can be used to shorten search times for finding the newly adopted AC and DC values within the reflected signal. Using the stress sensor unit, it can even be detected if there is no contact at all between the device and its user. Subsequently, the first LED and the detector can be switched off, thereby reducing power consumption significantly.

In a refinement the control unit further comprises a second output for providing a quality signal indicating a level of quality of the heart rate signal. The quality signal is provided as a function of the pressure signal and the reflected signal.

The quality signal basically indicates the validity of the heart rate signal. It shows the probability of the correctness of the heart rate signal. The quality signal can be used, for instance, for checking whether the provided heart rate signal which really reflects the user's heart rate, and not the frequency of his movements, in order to display only correct values of the heart rate.

In an embodiment the device further comprises a light barrier located between the first LED and the detector. The light barrier is prepared to prevent light from the light signal directly reaching the detector.

In a development the device further comprises a driver circuit coupled between the control unit and the first LED for driving the first LED depending on a driving signal. The driving signal is generated by the control unit as a function of the pressure signal.

Consequently, driving of the first LED is optimized by using information from the pressure signal in addition to driving of the first LED as known by the person skilled in the art.

In another refinement the detector comprises a light sensor and a first signal conditioning unit. The light sensor is configured to provide a detected signal as a function of the reflected signal. The signal conditioning unit is configured to provide a conditioned signal as a function of the detected signal and the pressure signal.

The pressure signal output by the stress sensor is used in conditioning signals within the detector. As described above, the detected signal comprises an AC value and a DC value which are provided by a current signal detected by, e.g., a photodiode of the light sensor. The current signal is converted into a voltage signal. Amongst others, an integrator is used within the first signal conditioning unit to calculate the DC value of the detected signal. By taking into account the pressure signal, a new DC value caused by a movement can be detected in a shorter time. As a consequence, the driving signal can be adapted such that the first LED is driven with a current which achieves an appropriate DC value in the detector.

In a development the device further comprises a gravity sensor unit for providing an acceleration signal indicative of a movement of the device's user. The heart rate signal is provided in the function of the acceleration signal.

According to this embodiment, the control unit uses the pressure signal, the conditioned reflected signal and the acceleration signal to calculate the user's heart rate.

Information of the acceleration signal can also be used in the quality signal indicating the probability of a correct heart rate signal.

In one embodiment the device is implemented in the form of a wrist watch.

In one embodiment a method for optical heart rate measurement using the portable device as described above comprises the following steps:
- generating a light signal with a predetermined wavelength,
- detecting a reflected signal depending on a reflection of the light signal off a blood vessel of a user of the device,
- sensing a pressure between the user and the portable device and therefrom providing a pressure signal,
- providing a heart rate signal as a function of the reflected signal and the pressure signal.

By additionally using information from the pressure signal, calculation of the heart rate signal can be accelerated.

In a development the method further comprises the step of providing a quality signal indicating a level of quality of the heart rate signal. The quality signal is provided as a function of the reflected signal and the pressure signal.

In another embodiment the method further comprises the steps:
- providing a detected signal as a function of the reflected signal by means of a light sensor,
- applying a signal conditioning to the detected signal using the pressure signal and providing a conditioned signal.

The pressure signal is additionally used in conditioning the detected signal of the light sensor.

The text below explains the invention in detail using exemplary embodiments with reference to the drawings. Components and circuit elements that are functionally identical or have the identical effect bear identical reference numbers. In so far as circuit parts or components correspond to one another in function, a description of them will not be repeated in each of the following figures.
- Figure 1: shows an embodiment of a portable device for optical heart rate measurement according to the proposed principle,
- Figure 2: shows another embodiment of a portable device according to the proposed principle,
- Figure 3: shows signal curves for the embodiment of Figure 2,
- Figure 4: shows a mechanical arrangement of a portable device according to the proposed principle,
- Figure 5: shows a side view of the arrangement of Figure 4,
- Figure 6: shows another mechanical arrangement of a portable device according to the proposed principle, and
- Figure 7: shows signals of a device for optical heart rate measurement according to the state of the art.

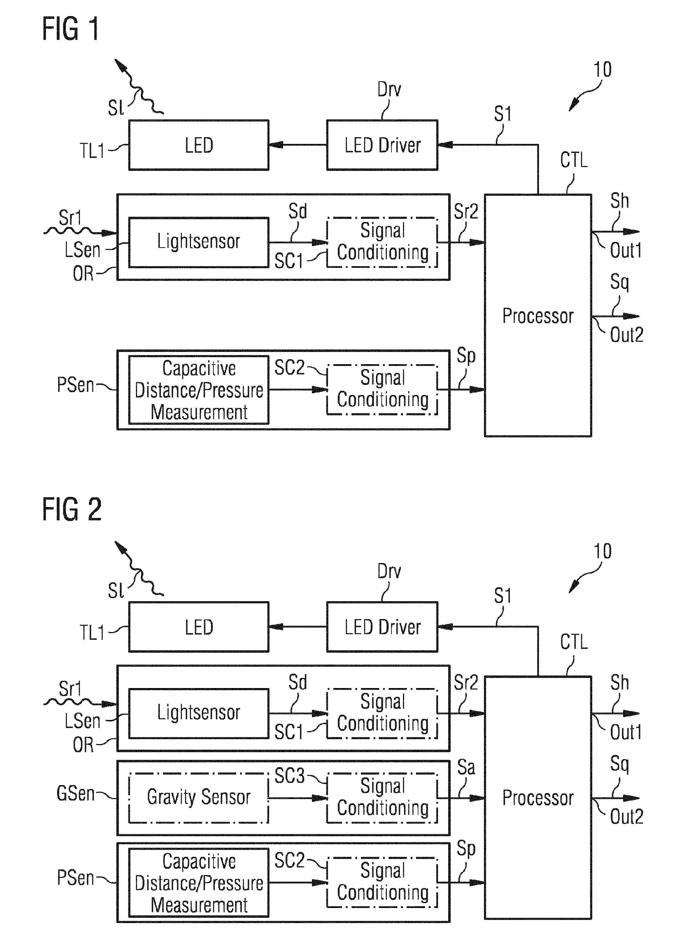

Figure 1 shows an embodiment of a portable device 10 for optical heart rate measurement according to the proposed principle. The portable device comprises a first light-emitting diode TL1, a driver circuit Drv, a detector OR, a stress sensor unit PSen, and a control unit CTL. The first LED TL1 is coupled via the driver circuit Drv to the control unit CTL. The detector OR and the stress sensor unit PSen are connected to the control unit CTL, as well. The control unit CTL has a first output Out1 and a second output Out2. The detector OR has a light sensor LSen and a first signal conditioning unit SC1.

The control unit CTL provides a driving signal S1 to the driver circuit Drv which causes the first light-emitting diode TL1 to generate a light signal Sl which has a predefined wavelength. The light signal Sl is reflected by a blood vessel of the device's 10 user and reaches the detector OR in the form of a reflected signal Sr1. In detail, the light sensor LSen within the detector OR receives the reflected signal Sr1 and therefrom provides a detected signal Sd. The detected signal Sd is conditioned, i.e. amplified and filtered within the first signal conditioning unit SC1 which therefrom provides a conditioned signal Sr2. Consequently, the conditioned signal Sr2 replicates a difference in mechanical expansion of a blood vessel which reflects the light signal Sl.

Concurrently, the stress sensor unit PSen measures a mechanical contact pressure between the device 10 and its user by means of a capacitive or a resistive sensor. This contact pressure is provided in the form of a pressure signal Sp to the control unit CTL. The control unit CTL also receives the conditioned signal Sr2. The control unit CTL uses the conditioned signal Sr2 and the pressure signal Sp to calculate a heart rate signal Sh which is provided at the first output Out1. Furthermore, the control unit CTL calculates a quality signal Sq which is provided at the second output Out2 as a function of the conditioned signal Sr2 and the pressure signal Sp.

By employing the stress sensor unit PSen, the heart rate signal Sh can be provided with higher precision and reflecting the heart rate in real time. The information of the quality Sq is improved at the same time by taking into account the information of the stress sensor unit PSen so that the indication of quality is more precise.

The quality signal Sq thereby gives a basic indication of whether the device 10 is adjusted correctly to the user with an appropriate pressure. Should this not be the case, the user could be informed by a corresponding request to readjust the device 10, the request being displayed on the device's screen, for instance.

Furthermore, the control unit CTL considers information from the pressure signal Sp in generation of the driving signal S1 for the driver circuit Drv controlling the first LED Tl1.

Usage of the stress sensor unit PSen also allows the detection of stray light which wrongfully reaches the detector OR whenever the device 10 is not attached tightly enough to the user. In this case light from an external light source may reach the detector OR in addition to the light from the first LED Tl1. Light from an external light source might also be driven with a frequency of roughly 60 Hz which is similar to a user's heart rate. Therefore, this stray light, when detected by the detector OR, might be interpreted wrongfully as the user's heart rate. Using the additional information of the stress sensor unit PSen can advantageously prevent this misinterpretation.

In a development the stress sensor unit PSen additionally comprises a second signal conditioning unit SC2. Signal conditioning operations as known by a skilled person are applied to the signal detected by the capacitive or resistive sensor within the stress sensor unit PSen.

In an exemplary implementation a value of the pressure signal Sp representing a momentary pressure between the device 10 and its user serves as a basis for selecting a new DC value for the conditioned signal Sr2 from a look-up table implemented for instance within the control unit CTL. Thereby, integrators used to calculate an AC component of the reflected signal Sr2 can be accelerated.

Figure 2 shows another embodiment of a portable device 10 for optical heart rate measurement according to the proposed principle. The embodiment of Figure 2 corresponds to the embodiment of Figure 1, except that in Figure 2 the device 10 additionally comprises a gravity sensor GSen which provides an acceleration signal Sa to the control unit CTL. The skilled person knows that the acceleration signal Sa is indicative of a point in time of a movement of the device's user. The gravity sensor GSen may also comprise a third signal conditioning unit SC3 which applies signal conditioning to a signal detected by the gravity sensor GSen in order to provide the acceleration signal Sa in a refined form.

In this embodiment, the control unit CTL provides the heart rate signal Sh and the quality signal Sq by using and combining information provided with the conditioned signal Sr2 of the detector OR, the pressure signal Sp of the stress sensor unit PSen and the acceleration signal Sa from the gravity sensor GSen. An algorithm running within the control unit CTL takes advantage of all these pieces of input information to calculate the signals provided at the first and second outputs Out1, Out2. In addition, driving the first LED Tl1 is further optimized by considering information provided via the acceleration signal Sa.

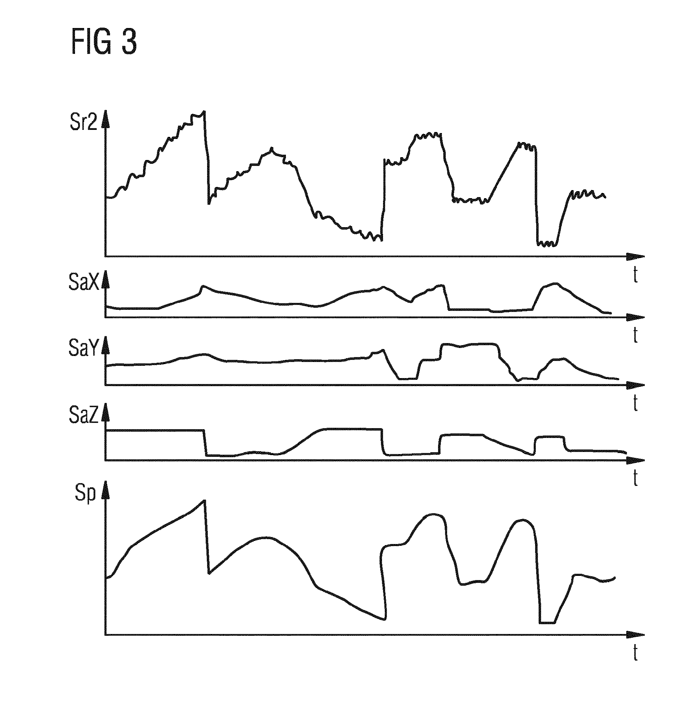

Figure 3 shows signal curves for the embodiment of Figure 2. All signal curves are depicted in relation to time t. The first line shows the conditioned signal Sr2. Second, third and fourth lines each show one component of the acceleration signal Sa which is depicted in its three components SaX, SaY and SaZ corresponding to the three dimensions of possible movement. The fifth line shows the pressure signal Sp.

Small ripples within the conditioned signal Sr2 represent the AC value of this signal which reflects the heart rate of the device's user. The outline of the conditioned signal Sr2 represents the DC value of the conditioned signal Sr2. Occurrence of movement of the device can be discerned from the acceleration signal components SaX, SaY and SaZ.

It can be seen that by taking into account information from the pressure signal Sp in conditioning the detected signal Sd of the light sensor LSen, the conditioned signal Sr2 is directly correlated to the movement of the device's user as calculated using the pressure signal Sp and the components SaX, SaY and SaZ of the acceleration signal Sa. A comparison with the signal output by a light sensor in the state of the art implementation as shown in Figure 7 reveals that the conditioned signal Sr2 of Figure 3 tracks the user's heart rate in real time even though movement occurs.

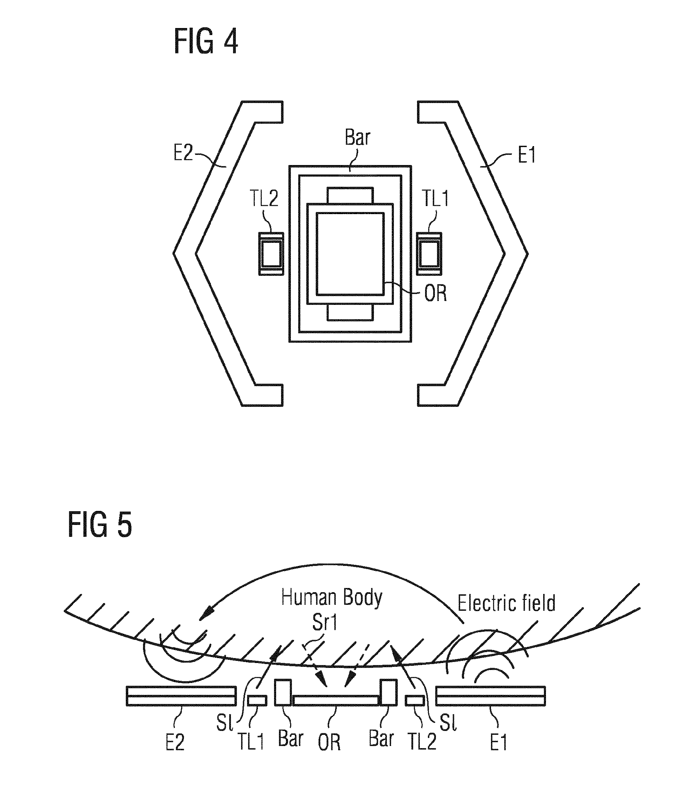

Figure 4 shows a mechanical arrangement according to an embodiment of the proposed device. The arrangement comprises a first and a second light-emitting diode Tl1, Tl2, a light barrier Bar, a detector OR and first and second electrodes E1, E2 which implement a stress sensor unit PSen as of the embodiments of Figure 1 or Figure 2. First and second electrodes E1, E2 are collectively operated as capacitive sensor in differential mode. This means that, for example, electrode E1 is operated as a transmitter and emits an electrical field, whereas second electrode E2 is operated as a receiver. The receiver electrode E2 detects changes in the electrical field caused by a difference in approximation of the device's user. In other words, the second electrode E2 detects different pressure between the device and its user.

In addition to the embodiments described above, this embodiment has a second light-emitting diode Tl2 which is also operated to emit light with a predetermined wavelength.

Figure 5 shows a side view of the mechanical arrangement of Figure 4. It can be seen that light signals Sl are emitted by first and second LEDs TL1, TL2. The detector OR receives the reflected signal Sr1 which is a function of a reflection of the light signal Sl within the human body. First electrode E1 generates an electric field. Changes within this electric field caused by the human body of the device's user are detected by means of the second electrode E2. The optical barrier Bar hinders light generated by first and second LEDs Tl1, Tl2 from reaching the detector OR directly.

Figure 6 shows another mechanical arrangement of a device according to the proposed principle. This arrangement is based on the arrangement of Figure 4 with two exceptions: first the form of first and second electrodes E1, E2 differs from the implementation as of Figure 4 and secondly, this arrangement has two pairs of electrodes. A first pair is formed of first and second electrodes, E1, E2, and a second pair is built up by third and fourth electrodes E3, E4. Each pair is operated as a capacitive sensor in differential mode, meaning that one of the electrodes is a transmitter generating an electric field and the second electrode of this pair is operated as a receiver which detects changes in capacitance in this field.

By using two pairs of electrodes, the position and the angle between the device with relation to its user can be detected with high precision so that the measurement of the human's heart rate can be accomplished with even higher accuracy. This also allows for a faster verification of valid measurement results.

Instead of a capacitive sensor, a resistive sensor can be used, for instance in the form of a piezo.

The invention is not limited to specific embodiments by the description on the basis of said exemplary embodiments but comprises any combination of elements of different embodiments. Moreover, the invention comprises any combination of claims and any combination of features disclosed by the claims.

### Reference list

- 10: portable device
- PSen: stress sensor unit
- TL1, TL2: light-emitting diode
- OR: detector
- CTL: control unit
- Sl: light signal
- Sr1: reflected signal
- Sp: pressure signal
- Sh: heart rate signal
- Sa: acceleration signal
- Sq: quality signal
- E1, E2, E3, E4: electrode
- Out1, Out2: output
- Bar: light barrier
- Drv: driver circuit
- LSen: light sensor
- SC1, SC2, SC3: signal conditioning unit
- GSen: gravity sensor
- Sr2: conditioned signal
- Sd: detected signal

## Claims

1. Portable Device (10) for optical heart rate measurement having
- a first light-emitting diode (TL1), LED, for generating a light signal (Sl) having a predetermined wavelength,
- a detector (OR) for detecting a reflected signal (Sr1), the reflected signal (Sr1) depending on a reflection of the light signal (Sl) off a blood vessel of a user of the device (10),
- a stress sensor unit (PSen) for providing a pressure signal (Sp) as a function of a pressure between the portable device (10) and its user, and
- a control unit (CTL) connected to the first LED (TL1), the stress sensor (PSen) and to the detector (OR), the control unit (CTL) having a first output (Out1) for providing a heart rate signal (Sh),
wherein the heart rate signal (Sh) is provided as a function of the reflected signal (Sr1) and the pressure signal (Sp) sensed by the stress sensor unit (PSen).

2. Device (10) according to claim 1,
wherein the wavelength of the light signal (Sl) is predetermined from a range of 525 to 950nm in accordance with the skin colour of the device's (10) user.

3. Device (10) according to claim 1 or 2,
wherein the stress sensor unit (PSen) comprises an electrode (E1) adapted to be operated as a capacitive sensor in loading mode.

4. Device (10) according to claim 1 or 2,
wherein the stress sensor unit (PSen) comprises two electrodes (E1, E2) adapted to be collectively operated as a capacitive sensor in differential mode.

5. Device (10) according to claim 1 or 2,
wherein the stress sensor unit (PSen) comprises a first and a second pair of electrodes (E1, E2, E3, E4), each of the two pairs being adapted to be operated as a capacitive sensor in differential mode, the pressure signal (Sp) being provided as a function of a first signal output by the first pair of electrodes (E1, E2) and a second signal output by the second pair of electrodes (E3, E4).

6. Device (10) according to claim 1 or 2,
wherein the stress sensor unit (PSen) comprises a resistive sensor.

7. Device (10) according to any of claims 1 to 6,
wherein the control unit further comprises a second output (Out2) for providing a quality signal (Sq) indicating a level of quality of the heart rate signal (Sh), wherein the quality signal (Sq) is provided as a function of the pressure signal (Sp) and the reflected signal (Sr1).

8. Device (10) according to any of claims 1 to 7,
further comprising a light barrier (Bar) located between the first LED (TL1) and the detector (OR), the light barrier (Bar) being prepared to prevent light from the light signal (Sl) directly reaching the detector (OR).

9. Device (10) according to any of claims 1 to 8,
further comprising a driver circuit (Drv) coupled between the control unit (CTL) and the first LED (TL1) for driving the first LED (TL1) depending on a driving signal (S1), said driving signal (S1) being generated by the control unit (CTL) as a function of the pressure signal (Sp).

10. Device (10) according to any of claims 1 to 9,
wherein the detector (OR) comprises a light sensor (LSen) and a first signal conditioning unit (SC1), wherein the light sensor (LSen) is configured to provide a detected signal (Sd) as a function of the reflected signal (Sr1) and wherein the signal conditioning unit (SC) is configured to provide a conditioned signal (Sr2) as a function of the detected signal (Sd) and the pressure signal (Sp).

11. Device (10) according to any of claims 1 to 10,
further comprising a gravity sensor unit (GSen) for providing an acceleration signal (Sa) indicative of a movement of the device's user, wherein the heart rate signal (Sh) is provided additionally in function of the acceleration signal (Sa).

12. Device (10) according to any of claims 1 to 11,
which is implemented in the form of a wrist watch.

13. Method for optical heart rate measurement using a portable device (10), the method comprising the following steps:
- generating a light signal (Sl) with a predetermined wavelength,
- detecting a reflected signal (Sr1) depending on a reflection of the light signal (Sl) off a blood vessel of a human under test,
- sensing a pressure between the human under test and the portable device (10) and therefrom providing a pressure signal (Sp),
- providing a heart rate signal (Sh) as a function of the reflected signal (Sr1) and the pressure signal (Sp).

14. Method according to claim 13,
further comprising
- providing a quality signal (Sq) indicating a level of quality of the heart rate signal (Sh), the quality signal being provided as a function of the reflected signal (Sr1) and the pressure signal (Sp).

15. Method according to claim 13 or 14,
further comprising
- providing a detected signal (Sd) as a function of the reflected signal (Sr1) by means of a light sensor (LSen),
- applying a signal conditioning to the detected signal (Sd) using the pressure signal (Sp) and providing a conditioned signal (Sr2).
